# EUROPEAN PATENT SPECIFICATION

(11) **EP 1 946 109 B1**
(45) Date of publication and mention of the grant of the patent: **20.01.2010**
(21) Application number: 06778497.5
(22) Date of filing: 10.08.2006
(51) Int. Cl.: G01N 33/564, C07K 7/08

(54) **METHOD FOR DETECTING AUTOANTIBODIES FORMED IN RHEUMATOID ARTHRITIS**
VERFAHREN ZUM NACHWEIS VON BEI RHEUMATOIDER ARTHRITIS GEBILDETEN AUTOANTIKÖRPERN
MÉTHODE DE DÉTECTION D'AUTO-ANTICORPS FORMÉES DANS LA POLYARTHRITE RHUMATOIDE

(30) Priority: 11.08.2005 FI 20050814
(43) Date of publication of application: 23.07.2008
(73) Proprietor: Procollagen OY, 90500 Oulu (FI)
(72) Inventor: KOIVULA, Marja-Kaisa, 90014 Oulun Yliopisto (FI); RISTELI, Juha, FI-90500 Oulu (FI)
(74) Representative: Sundman, Patrik Christoffer
(86) International application number: PCT/FI2006/000275
(87) International publication number: WO 2007/017556

(56) References cited:
- WO-A-00/79284
- WO-A2-03/032909
- WO-A2-03/050542
- WO-A2-03/068919
- WO-A2-2004/078098
- WO-A2-2004/087747
- BURKHARDT H ET AL: "Humoral immune response to citruillinated collagen type II determinants in early rheumatoid arthritis" ANNALS OF THE RHEUMATIC DISEASES, vol. 64, no. Suppl. 3, July 2005 (2005-07), page 164, XP008072566 & ANNUAL EUROPEAN CONGRESS OF RHEUMATOLOGY; VIENNA, AUSTRIA; JUNE 08 11, 2005 ISSN: 0003-4967
- KOIVULA M -K ET AL: "Are there autoantibodies reacting against citrullinated peptides derived from type I and type II collagens in patients with rheumatoid arthritis?" ANNALS OF THE RHEUMATIC DISEASES, vol. 64, no. 10, October 2005 (2005-10), pages 1443-1450, XP008072561 ISSN: 0003-4967
- KOIVULA MARJA-KAISA ET AL: "Sensitive immunoassays for the autoantibodies reacting against citrullinated carboxy-terminal telopeptides of type I and type II collagens in patients with rheumatoid arthritis" CLINICAL CHEMISTRY AND LABORATORY MEDICINE, vol. 43, no. 12, December 2005 (2005-12), pages 1400-1405, XP008072567 ISSN: 1434-6621

## Description

The invention relates to a method for detecting autoantibodies found in patients with rheumatoid arthritis (RA).

Rheumatoid arthritis is a common autoimmune disease affecting about 1 % of the world population. It is difficult to recognize in the early stages, and the disease subsequently proceeds to irreversibly damage in the joints by destroying the cartilage and leading to the erosion of the periarticular bones.

Citrulline is an amino acid that is post-translationally formed from arginine in peptides or proteins by the enzyme peptidylarginine deiminases (PADs). This structure has attracted considerable attention in rheumatology, as several autoantibodies in RA patients are directed to proteins that contain citrulline. The first such autoantibody was the antiperinuclear factor (APF) described by Neinhuis and Mandema as early as 1964 (Ann Rheum Dis 1964; 23: 302-305). This antigen is present in the keratohyaline granules surrounding the nucleus of human buccal mucosa cells. In 1979, Young et al (BMJ 1979; 2: 97-99) discovered an antikeratin antibody (AKA) by indirect immunofluorescence using sections of rat oesophagus. Schellekens et al (J Clin Invest 1998; 101: 273-281) and Girbal-Neuhauser et al (J Immunol 1999; 162: 585-594) later showed out independently that both APF and AKA specifically bind (pro)filaggrin, which contains citrulline residues. These autoantibodies are more specific for RA than certain traditional autoantibodies, e.g. the rheumatoid factor, which constitutes one of the classification criteria proposed by the American College of Rheumatology (Arnett et al., Arthritis Rheum 1988; 31: 315-324). On the other hand, it has been speculated that, because of their specificity, the antibodies to citrullinated proteins could be involved in the aetiology and/or pathogenesis of RA (van Boekel et al. Arthritis Res 2002; 4: 87-93). However, the fact that the production of anti-citrullinated filaggrin antibodies precedes disease onset contradicts the simple hypothesis that citrullination and/or anti-citrullinated filaggrin antibodies are pathogenic on their own (Yamada et al. Front Biosci 2005; 10: 54-64). E.g. deep destruction of cartilage and erosion of bone in periarticular tissue cannot be induced by these autoantibodies.

The bindings of autoantibodies to citrullinated proteins can be detected by several methods. The immunostaining methods used at first were not suitable for routine use in the clinical laboratory, but the introduction of enzyme-linked immunosorbent assays (ELISA), later with cyclic, citrullinated peptides as antigens (anti-CCPs), has greatly simplified the determination of such antibodies with good reproducibility (Schellekens et al. et al: Arthritis Rheum 2000; 43: 155-163). The presence of anti-CCP antibodies has been reported to predict the development of RA in apparently healthy individuals (Rantapää-Dahlqvist et al. Arthritis Rheum 2003; 48: 2741-2749 and Nielen et al. Arthritis Rheum 2004; 50: 380-386) and the progression of undifferentiated arthritis to RA (van Gaalen et al: Arthritis Rheum 2004; 50: 709-715). Possible clues concerning the pathogenetic role of these antibodies include an association of citrullination with apoptosis, the appearance of anti-CCP antibodies before the occurrence of clinical symptoms and their specificity for RA and the suggested genetic risk factor that leads to increased citrullination associated with RA (see van Gaalen FA et al. Arthritis Rheum 2004; 50: 709-715).

There are no anti-CCP antigens in joints, which means that any autoantibodies reacting with these antigens most probably only reflect an immunological cross-reaction (Yamada et al. Front Biosci 2005; 10: 54-64). Possible candidates for the original immunogen include citrullinated α and β chains of fibrin in synovial tissue (Masson-Bessiere et al: J Immunol 2001; 166: 4177-4184) and the Sa antigen, identified as citrullinated vimentin (Vossenaar at al. Arthritis Res There 2004; 6:R142-R150).

It has long been supposed that autoimmunity against collagens might be involved in the pathogenesis of RA. However, the evidence is only circumstantial. In certain animal species, e.g. rats, mice, rabbit and monkeys, immunisation with type II collagen results in the development of polyarthritis that resembles human RA. Type II collagen is post-translationally modified, and some of these modifications (e.g. glycosylations of lysine at position 264) may be recognised by T cells (Holmdahl et al. Ageing Res Rev 2002; 1:135-147). Anti-collagen antibodies are not formed only against cartilage collagen, but also against bone (type I collagen) and soft tissue collagens (e.g. type III and V collagens) (Stuart et al. Arthritis Rheum 1983; 26: 832-840). Antibodies against denatured collagens are more frequent and present in higher concentrations in RA sera than antibodies against native collagens (Nijenhuis et al. Clin Chem Acta 2004; 350: 17-34 and Nomura K: Arch Biochem Biophys 1992; 293: 362-369). These anti-collagen antibodies are by no means specific for RA, and their formation could be secondary to the destruction of connective tissues rather than a cause of the disease.

Several years ago many papers were published dealing with the treatment of rheumatoid arthritis with orally administered type II collagen (Trentham et al. Science 1993;262:1727-1730). However, the experiments did not show any benefit (Cazzola et al. Clin Exp Rheumatol 2000;18:571-577) or only small disease improvement of RA (Choy et al. Arthritis Rheum 2001;44:1993-1997), and interest in this hypothesis has since been lost.

Burckhardt et al. (Eur J. Immunol 2005. 35: 1643 -1652) tested the hypothesis, whether collagen II (CII) might be a substrate for structural modification by PAD enzymes, which could result in a breakage of self tolerance to this cartilage-specific auto-antigen. The results gave evidence that PAD2 (PAD isotype 2) catalyzes conversion of arginine to citrulline in a CII region of immunodominance for autoantibody formation and showed the presence of circulating autoantibodies that specifically bind to the citrullinated triple helical collagen peptide CII amino acid residues 359-369 in early RA patients. According to Burkhardt et al. their results indicate that autoimmune recognition of citrullinated collagen is a frequent event in early RA. The authors further noted that the IgG antibody to citrullinated CII correlates with the occurrence of anti-CCP autoantibodies, which are used as early markers of RA.

Suzuki et al. (Bioch Biophys Res Commun 2005;33:418-426) found out that human type I collagen could be one of the autoantigens, using a RA synoviocyte cDNA library by immunoscreening. They tested the hypothesis, whether type I and type II collagen could be used as substrates for citrullination. In contrary to the findings of the previous paper (Burckhardt et al. Eur J Immonul 2005; 35:1643-1652), there were no specific antibodies against citrullinated type II collagen in RA, but antibodies were found against citrullinated type I collagen.

The anti-CCP autoantibodies, although very specific for rheumatoid arthritis, are probably harmless, since they cannot be responsible for the destruction of cartilage and periarticular bone. The first assay published for anti-CCP (mark 1) detects citrullinated filaggrin, which has a sequence of -STXG-, where X is citrulline (Schellekens et al: Arthritis Rheum 2000;43:155-163). The second assay published for anti-CCP (mark 2) is based on several peptide sequences that have been selected from randomly generated synthetic peptides on the basis of discriminating between RA and control sera (Zendman A et al, Rheumatology 2006,45:20-5 and Kinloch AJ et al, Expert Rev Clin Immunol 2006; 2; 365-375). The sequences have not been published, but they are not related to either filaggrin or any other proteins found in human bodies.

Lundberg et al. (Arthritis Res Ther 2005;7:R 458-R467) examined the responses of rat T and B cells to citrullinated rat serum albumin (Cit-RSA) in comparison with those of unmodified rat serum albumin (RSA). It was found out that Cit-RSA leads to breakdown of immunological tolerance, since antibodies were produced against both Cit-RSA and RSA antigens. However, RSA alone did not induce any antibodies. Citrullinated collagen II (Cit-CII) induced arthritis with higher incidence and earlier onset than did the native counterpart. The amount of citrullinated proteins and the enzyme PAD4 correlated, according to Lundberg et al, with severity of inflammation and were not detectable in healthy joints.

Examples of known collagen amino acid sequences can be found in databases, for example in databases EMBL-EBI, AC Q8N473, 01-OCT-2002, UniProtKB/TrEMBL, Alpha 1 type I collagen, preprotein, EMBL-EBI, AC Q7Z5S6, 01-OCT-2003, UniProtKB/TrEMBL, Alpha 2 type I collagen, EMBL-EBI, AC Q96IT5, 01-DEC-2001, UniProtKB/TrEMBL, COL2A1 protein (Collagen type II alpha 1).

An assay for the cross-linked carboxyterminal telopeptide of type I collagen (ICTP) has been shown to reflect increased type I collagen degradation in some pathological conditions as rheumatoid arthritis. The antigenic determinant recognized by the ICTP assay was shown by Sassi M.-L. et al. (Bone, April 2000; 26(4):367 -373 to reside within the hydrophobic phenylalanine-rich regions of the carboxyterminal telopeptides of the two α1 chains of human type I collagen (AGFDFSFL in human type I collagen). Nordic Bioscience diagnostics CrossLaps ® ELISA http://www.nbdiagnostics.com assays can be used for assessment of bone resorption. It detects collagen type I fragments generated during osteoclastic bone resorption and employs monoclonal antibodies recognizing C-telopeptide fragments of collagen type I α1 chains containing the epitope Glu-Lys-Ala-His-Asp- β -Gly-Gly-Arg in an isomerised form.

There are several patent publications, which disclose peptides suggested for use in diagnosing rheumatoid arthritis. International Patent Publication WO 95/08115 describes a method for determining collagen fragments in a body fluid. The method uses synthetic peptides derived from collagen and containing potential sites for cross-linking. Also in US 6,355,442 a method is disclosed for determining collagen fragments. International Patent Publication WO 03/050542 describes a method for detecting autoantibodies from patients suffering from rheumatoid arthritis. The method comprises the use of a peptide unit comprising XG motif and XnonG, wherein X is citrulline residue or an analogue thereof, G is the amino acid glycine and nonG is an amino acid other than glycine. International Patent Publication WO 2004/078098 discloses citrullinated peptides having increased affinity to a MHC class II molecules with the shared epitope. Such peptides are suggested to be used in diagnosing rheumatoid arthritis. International Patent Publication WO 2004/087747 discloses linear citrullinated synthetic peptides and multiple antigen peptides comprising them. The peptides are also suggested to be used in diagnosing rheumatoid arthritis.

It is an aim of the present invention to provide an improved method for diagnosing rheumatoid arthritis.

### SUMMARY OF THE INVENTION

One object of the present invention is a method for detecting autoantibodies associated with rheumatoid arthritis. The method can be used in the diagnosing of the disease.

The method is based on the finding that in patients having rheumatoid arthritis autoantibodies were formed against parts of collagen peptides. In particular, such parts of collagen peptides are telopeptides, more specifically the carboxyterminal telopeptides. It was found that the C-telopeptides comprised peptide sequences, which were selected from the group of -YYXA, -FYXA, and -YMXA, where X stands for arginine converted to citrulline. -YYXA was found in α1 chain of type I collagen, -FYXA was found in α2 chain of type I collagen and -YMXA was found in α1 chain of type II collagen. These autoantibodies were different from those detected with the sequence based on filaggrin. The anti-CCP method suggested in the prior art (Schellekens et al: Arthritis Rheum 2000;43:155-163) could therefore not detect the correct peptide sequences associated with rheumatoid arthritis.

One further object of the invention is peptides and pharmaceutical compositions for treating RA. It was found in the present invention that parts of citrullinated collagens can induce immunological tolerance against potentially harmful citrullination of collagens. RA patients could thus be treated by administering them parts of citrullinated collagens. In particular, such parts of collagen were from telopeptides, more specifically the carboxyterminal telopeptides. It was found that the C-telopeptides comprised peptide sequences, which were selected from the group of -YYXA, -FYXA, and -YMXA, where X stands for arginine converted to citrulline.

The prior art publications do not disclose studies of the role of collagen telopeptides in autoantibody formation in patients having RA. The reason is that such autoantibodies have mainly been tested by using collagen preparations rendered soluble by pepsin digestion. This protease removes the carboxy telopeptides of collagens and the authors of the prior art publications thus could not make any observances of telopeptides.

Furthermore, the present invention can be used to monitor patients with RA treated with peptides of the present invention.

The assays developed in this study can be used both to detect the autoantibodies and diagnose RA in patients and to monitor patients with RA treated with peptides of the present invention.

Anti-CCP method functions well in diagnosis of rheumatoid arthritis. However, the autoantibodies detected by the method cannot be responsible for cartilage and bone degradation. Therefore, it is important to recognize those patients who need the most aggressive treatment with drugs.

Many drugs used for treating patients having or suspected to have rheumatoid arthritis cause several side effects. Therefore, the drugs cannot be given to all patients. If a patient is shown to have antibodies against citrullinated collagens, this is a reason to treat the patient in a most aggressive manner. The most recent drugs are also very expensive, which prevents their broad use. By using the method of the invention those patients who need the most effective treatment can be recognized and the treatment can be directed to the right groups of patients.

### BRIEF DESCRIPTION OF THE DRAWING

Figure 1 shows the sequences of and locations of the chosen peptides in the primary structures of human type I and II collagens.
Figure 2. A serum sample was tested on three different ELISAs: binding of citrullinated carboxytelopeptides of the α1 chain of type I (A) and II collagen (B) and reaction in the anti-CCP 2 assay (C).

### DETAILED DESCRIPTION OF THE INVENTION

"Rheumatoid arthritis" (RA) is a prevalent autoimmune disease characterized by synovial inflammation and pannus formation, which can lead to cartilage and bone degradation.

Three classes of human collagen have been described. The first group contains several collagen types, for example types I, II, III and V. From these, type I collagen accounts for more than 90 % of the organic matrix bone. Type II collagen is an abundant molecule in articular cartilage. The collagen types I and II are synthesized as procollagens, containing N-terminal and C-terminal propeptide sequences, which are attached to the core collagen molecules. When the propeptides are removed, the remaining core of the collagen molecules consists of a triple-helical domain having short terminal telopeptide sequences, which are non-helical.

By the term "autoantibodies" is meant antibodies, which are formed against arginine containing peptides or their citrullinated forms in a patient having rheumatoid arthritis. These autoantibodies are formed in association to cartilage and bone degradation.

In this invention it has been found that in particular the carboxyterminal telopeptides comprise sequences which can be used in detection of autoantibodies. Such sequences have been found specifically from the α1 chain of type I collagen, the α2 chain of type I collagen and the α1 chain of type II collagen. Furthermore, it has been found that peptide sequences comprising sequences from these carboxyterminal parts can be used in detection of autoantibodies, if the last arginine residue in the peptide sequence is converted to citrulline.

The length of the carboxyterminal telopeptide of the α1 chain of type I collagen is 26 amino acids. The length of the carboxyterminal telopeptide of the α2 chain of type I collagen is 17 amino acids and the length of the carboxyterminal telopeptide of the α1 chain of type II collagen is 27 amino acids.

The present invention encompasses in particular peptides which comprise the amino acid sequence of the C-terminal parts of the carboxyterminal telopeptides. Of special interest is that part of the telopeptide(s) which can induce the formation of an autoantibody complex when contacted with a biological fluid sample under suitable conditions for the formation of immunological complexes.

The peptide preferably ends with a sequence selected from the group comprising-YYXA, -FYXA and -YMXA, wherein X is citrulline.

According to the most preferred embodiment of the invention, the peptide comprises or has a sequence selected from the group comprising EKAHDGGRYYXA (SEQ ID NO:1), YDFGYDGDFYXA (SEQ ID NO:2), and EKGPDPLQYMXA (SEQ ID NO:3), where X is citrulline.

The expression "peptide sequence comprises a sequence derived from a carboxyterminal telopeptide" means that the peptide sequence comprises a sequence which is the same as that of the carboxyterminal telopeptide or has such amino acid changes that the peptide sequence still functions in the same manner. Preferably, the peptide sequence comprises a sequence which is the same as that of the carboxyterminal telopeptide.

The length of the peptide sequences of the present invention are preferably 10 to 30 amino acids, more preferably 12 to 27 amino acids. Preferably they are at least 12 amino acids, more preferably at least 17 amino acids, most preferably at least 26 amino acids.

The peptide sequences can be isolated from tissue collagens and the arginine can be converted to citrulline *in vitro.* Purification can be achieved by protein purification methods well known for a person skilled in the art. Protein purification methods are, for example, chromatography methods, such as gel-filtration, ion-exchange and immunoaffinity, high-performance liquid chromatography (HPLC), high-performance chromatofocusing and hydrophobic interaction chromatography, or precipitation, in particular immunoprecipitation.

Arginine can be post-translationally modified to citrulline through deimination. This process is catalyzed by the enzyme peptidylarginine deiminase (PAD). The deimination process can be carried out *in vitro.*

The peptide sequences can also be synthesized by methods well known for a person skilled in the art. Such methods are for example chemical synthesis methods, such as solid phase synthesis (Merrifiel, 1964. J. Am. Chem. Assoc. 65:2149; J. Amer. Chem. Soc. 85:2149 (1963) and Int. J. Peptide Protein Res. 35:161-214 (1990)) or the synthesis can be done in homogenous solution (Methods of Organic Chemistry, E. Wansch (Ed. Vol. 15 pts. I and II. Thieme, Stuttgart (1987).

The peptide sequences can also be prepared by recombinant DNA techniques well known for a person skilled in the art. The deimination process can be carried out *in vitro* by using the enzyme peptidylarginine deiminase (PAD).

The body fluid sample is preferably a serum sample or a sample of another biological fluid, such as a synovial fluid sample.

The peptide sequences of the invention may be labelled to with a label in order to facilitate their detection in various assays. Examples of such labels are for example a radioactive label, a fluorescent label, a luminescent label, a lanthanide or an enzyme.

The immunologic reaction between the peptide sequence and the biological fluid sample may be carried out in a solid phase. The peptides may be covalently or non-covalently coupled to a solid carrier, such as a microphere of gold or polystyrene, a slide, a chip or a wall of a microtitre plate. The peptide sequences can be bound directly or indirectly, for example via streptavidin, to the test tube or plate.

Alternatively, the immunologic reaction between the peptide sequence and the biological fluid sample may be carried out in a liquid phase and the peptide sequence may be bound directly or indirectly, for example via streptavidin, to solid particles, such as magnetic particles.

The present invention encompasses also a pharmaceutical composition. The pharmaceutical composition comprises an effective amount of at least one of the peptide sequences of the invention. The pharmaceutical composition is in biologically compatible form suitable for administration *in vivo* for patients. Such composition should not have any toxic effects. A pharmaceutical composition comprises an effective amount of peptide sequences and pharmaceutically acceptable additives. By additives is meant pharmaceutically acceptable carriers, such as gelatin, dextrin, pectin, agar, oil, saline, sucrose, lactose, calcium phosphate and water, stabilizers, such as carbohydrates (for example mannitol, starch, sucrose, dextrin, glucose and sorbitol), proteins (for example casein and albumin), and buffers. The pharmaceutical composition may comprise also one or more adjuvants.

"Effective amount" is an amount effective at dosages and for periods of time, necessary to achieve the desired result in a human. The desired result is the immune response in a human and may depend on the age, weight, health, sex or disease state. The pharmaceutical composition may be administrated in the amount of 1 - 50 µg/day. Preferably the amount is 1 - 25 µg/day. The amount of administration may depend on the route of administration, time of administration and may be varied depending on the immune response of an individual.

Suitable administration routes are subcutaneus injections, intramuscular injections, intravenous injections or intraperitoneal injections, oral and intranasal administration. Most suitable routes are oral administration or injection.

A kit comprises at least one peptide according to the invention. The test kit can be used in the diagnostic or monitoring method according to the invention.

According to a preferred embodiment of the invention the autoantibodies in a biological fluid sample can be detected by using a solid substrate, such as microtitration plate, coated with the citrullinated peptides of the invention. A sample of a biological fluid is placed in contact and incubated with the peptides absorbed on the substrate. The non-bound material of the biological sample is removed by washing. An indicator antibody capable of binding any antibodies present in the citrullinated peptide/ anti-citrullinated peptide antibody complex is added to the solid substrate. The indicator may be a anti - human IgG immunoglobulin. The presence of the citrullinated peptide/ anti-citrullinated peptide antibody/ indicator antibody complex on the solid substrate is detected. The detection may be based for example on chemiluminesence and the emitted light measured by luminometer.

According to another preferred embodiment of the invention the assay is carried out by ELISA method, where the indicator antibody is conjugated to an enzyme. Suitable enzymes are for example enzymes which can be detected with the use of a chromogenic substrate. Such enzymes are for example alkaline phosphatase, horseradish peroxidase and β-galactosidase. The chromogenic substrate gives rise to a coloured product as a result of the reaction with the enzyme. The coloured product can be detected spectrofotometrically.

The presence of autoantibodies in a biological fluid sample can be detected as an immunological reaction with the peptides of the invention by using for example an ELISA method or a chemiluminesence immunoassay. The abundance of autoantibodies in a biological fluid can be measured as the intensity of the absorbance by spectrofotometer or the light of a chemiluminesence reaction quantified by luminometer. The treatment of RA with the peptides of the invention can be monitored by comparing the amount of autoantibodies in a patient before and after the treatment.

Since there are also antibodies against normal type I and type II collagens in rheumatoid arthritis, for detecting specific antibodies against citrullinated forms two assays should be performed using both arginine and citrulline containing peptides of the type I or type II collagen carboxytelopeptides. The absorbance of arginine containing peptides is subtracted from the absorbance of citrulline containing peptide or the binding ratio of citrulline and arginine containing peptides is calculated. Alternatively it is possible to use only the assay version with the citrullinated peptide, performing it both with and without addition of the same or similar peptide in soluble form as inhibitor of the immunological binding.

Examples are given below to illustrate the present invention in further detail.

### EXAMPLE 1

### ELISA assay

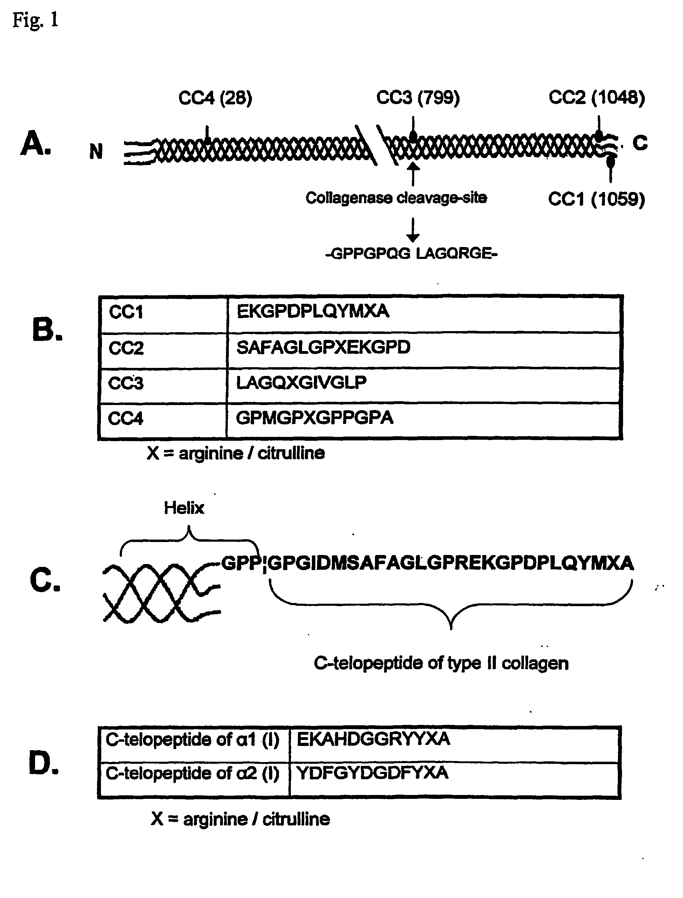

The serum samples from 120 patients with RA were obtained from the Division of Rheumatology of Oulu University Hospital. The controls consisted of 81 sera from healthy people matched for age and sex. Six pairs of biotinylated peptides were synthesised by NeoMPS (Strasbourg, France). Figure 1 shows the sequences and locations of the chosen peptides in the primary structures of human type I and II collagens.
Fig 1A-C show the localization of the peptides CC1 - CC4 in type II collagen and their sequences; the numbers in brackets refer to arginine residues: CC1 (1059), CC2 (1048), CC3 (799) and CC4 (28). Collagenase cleavage site is shown by arrow in Fig. 1A -GPPGPQG|LAGQRGE- (SEQ ID NO:9). Fig. 1B shows the sequences CC1 EKGPDPLQYMXA (SEQ ID MO:3), CC2 SAFAGLGPXEKGPD (SEQ ID NO:6),CC3 LAGQXGIVGLP (SEQ ID NO:7) and CC4 GPMGPXGPPGPA (SEQ ID NO:8); X is arginine/citrulline. Fig. 1C shows detailed structure of the carboxy terminal telopeptide of type II collagen (GPP belongs to the helix); C-telopeptide of type II collagen has the sequence GPGIDMSAFAGLGPREKGPDPLQYMXA. The sequence GPPGPGIDMSAFAGLGPREKGPDPLQYMXA is shown in the sequence listing as SEQ ID NO: 10. The 12 carboxy terminal amino acids represent the peptide CC1.
Fig 1D shows the sequences of the carboxyterminal telopeptides of the α1 (EKAHDGGRYYXA; SEQ ID NO:1) and α2 (YDFGYDGDFYXA; SEQ ID NO:2) chains of type I collagen. One member of each pair contained the arginine predicted by the respective gene, whereas in the other member of the pair this was replaced by citrulline.

The biotinylated peptides were coupled to streptavidin-coated 96-well assay plates (BioBind Assembly, Thermo Labsystems Oy, Vantaa, Finland) at a concentration of 10 µg/well. The coupling was performed at room temperature, pH 7.5, for two hours. The streptavidin-coated wells had been blocked by the manufacturer to prevent unspecific binding.

The sera to be tested were diluted in assay buffer (10mM Tris-HCl, 350mM NaCl, 1% BSA, 1% [vol/vol] Triton X-100, 0.5% [wt/vol] Na-deoxycholate, 0.1% SDS; pH 7.6) supplemented with 1% rabbit serum and incubated for an hour at room temperature. After washing (3 times with PBS/0.05% [vol/vol] Tween-20), 100 µl of anti-human IgG conjugated to peroxidase (Product # 31412, Pierce, Rockford, IL, USA) diluted 1:7500 in EIA buffer (20mM Tris-HCl, 150mM NaCl, 0.1% BSA, 0.05% Tween-20, pH 7.5) was added. After incubation for an hour at room temperature, the plates were washed (3 times with PBS/Tween-20). The bound antibodies were detected with 3,3'-5,5'-tetramethylbenzidine (Sigma-Aldrich, St.Louis, MN, USA) as a substrate (0.01 mg/100 µl per well in 100 mM sodium acetate trihydrate, 1.5 mM citric acid monohydrate, 0.0015 % H₂O₂). After 30 minutes, the reaction was stopped by adding 100 µl 2M sulphuric acid/well. The plates were read at a wavelength of 450 nm in a Victor² instrument (Wallac, Turku, Finland) and calculated by Multicalc (Wallac). All the sera were tested in duplicate. The coefficients of variation were generally less than 10 %.

When the single peptides were analysed directly, 42 % - 53 % of the patients with RA showed increased binding especially of the peptides derived from type II collagen (table 1).

**Table 1. Binding of the peptides to human sera in ELISA.**

| | | Controls mean ± SD absorbance | Controls over mean + 2 SD number | RA patients over mean + 2 SD number |
|---|---|---|---|---|
| C-telopeptide of α1(I) | | | | |
| | Arginine peptide | 0.288 ± 0.142 | 4 / 81 | 6 / 120^{ns} |
| | Citrulline peptide | 0.314 ± 0.117 | 2 / 81 | 24 / 120^{***} |

| C-telopeptide of α2(I) | | | | |
|---|---|---|---|---|
| | Arginine peptide | 0.692 ± 0.326 | 3 / 81 | 22 / 120^{***} |
| | Citrulline peptide | 0.763 ± 0.356 | 3 / 81 | 12 / 120^{**} |

| CC1 = C-telopeptide of α1(II) | | | | |
|---|---|---|---|---|
| | Arginine peptide | 0.181 ± 0.077 | 2 / 81 | 54 / 120^{***} |
| | Citrulline peptide | 0.155 ± 0.065 | 2 / 81 | 35 / 120^{***} |

| CC2 | | | | |
|---|---|---|---|---|
| | Arginine peptide | 0.162 ± 0.053 | 3 / 81 | 50 / 120^{***} |
| | Citrulline peptide | 0.135 ± 0.054 | 1 / 81 | 25 / 120^{***} |

| CC3 | | | | |
|---|---|---|---|---|
| | Arginine peptide | 0.182 ± 0.073 | 2 / 81 | 63 / 120^{***} |
| | Citrulline peptide | 0.181 ± 0.067 | 2 / 81 | 32 / 120^{***} |

| CC4 | | | | |
|---|---|---|---|---|
| | Arginine peptide | 0.179 ± 0.070 | 3 / 81 | 60 / 120^{***} |
| | Citrulline peptide | 0.184 ± 0.071 | 2 / 81 | 22 / 120^{***} |

| | | | | |
|---|---|---|---|---|
| ^{ns} not significant, ^{**} p < 0.01 and ^{***}p < 0.001 compared with controls | | | | |

When each peptide pair was being tested, the means (absorbance value of citrullinated peptide minus that of arginine peptide) and variances of the differences of the control samples were -0.026 (SD 0.032) for CC1, -0.027 (0.029) for CC2, -0.001 (0.026) for CC3 and +0.005 (0.020) for CC4. There was no specific binding to the citrullinated forms of the CC3 and CC4 of the RA sera. However, peptide CC2 and especially peptide CC1 in the RA patients showed differences, since two sera in the peptide CC2 group and 12 sera in the peptide CC1 group bound more citrullinated peptide than normal peptide. For the α1 chain of type I collagen, the means (absorbance value of citrullinated peptide minus that of arginine peptide) and the variances of the differences of the control samples were around +0.026 (0.074). With the C-telopeptide of the α2 chain from type I collagen, the means and variances of the differences between the control samples were +0.071 (0.252). 20 RA sera bound the citrullinated carboxytelopeptide from the α1 chain of type I collagen (α1(I) telopeptide) more strongly than the respective arginine peptide.

We found that the peptide CC1 and, to a lesser extent, the peptide CC2 specifically bound autoantibodies against citrullinated proteins. These results indicate that the arginine near the carboxyterminal end susceptible to the action of PAD enzyme.

### EXAMPLE 2

### Chemiluminesence assay

The measurements were done with two-site chemiluminescence immunoassays, which detect IgG antibodies against the synthetic C-telopeptides of the α1 chains of human type I and II collagens (EKAHDGGRYYRA (SEQ ID NO:4), and EKGPDPLQYMRA (SEQ ID NO:5), or EKAHDGGRYYXA (SEQ ID NO:1), and EKGPDPLQYMXA (SEQ ID NO:3), respectively, where X stands for citrulline, from NeoMPS, Strasbourg, France). The serum samples were first diluted 1:10 in a buffer containing 10mM Tris-HCl, 350mM NaCl, 1% BSA, 1% [vol/vol] Triton X-100, 0.5% [wt/vol] Na-deoxycholate, 0.1% SDS; pH 7.6 and incubated with suitable concentrations of one of the above peptides (in biotinylated form) and streptavidin-coated magnetic particles for 10 minutes at 37°C. The unbound biotinylated antigen and antibodies were separated from the complex bound to the magnetic particles by aspiration of the reaction mixture and subsequent washing. Thereafter, acridinium-labeled anti-human IgG antibodies were added to the reaction mixture followed by another 10-minute incubation to produce the sandwich complex. The unbound label was separated by aspiration of the reaction mixture and subsequent washing. To the washed magnetic particles with the complex we injected the triggers 1 and 2, initiating the chemiluminescence reaction. The trigger 1 solution contained hydrogen peroxide in diluted acid, and the trigger 2 solution contained diluted sodium hydroxide.

Serum samples from 120 patients with early RA were obtained from the Division of Rheumatology of Oulu University Hospital. The controls consisted of 81 sera from age- and sex-matched healthy persons.

In 44 RA patients out of 120, the sera showed increased binding of citrullinated synthetic C-telopeptide derived from the α1 chain of type I collagen (p = 0.003 compared to controls). For a corresponding C-telopeptide pair from the α1 chain of type II collagen, 35 patients' sera bound the citrullinated peptide more strongly than the arginine peptide, but the difference compared to the controls was not significant (p = 0.074). The correlation between the two carboxytelopeptides was r = 0.473 (p < 0.001).

### EXAMPLE 3

### The specificity of autoantibodies in sera of rheumatoid arthritis patients

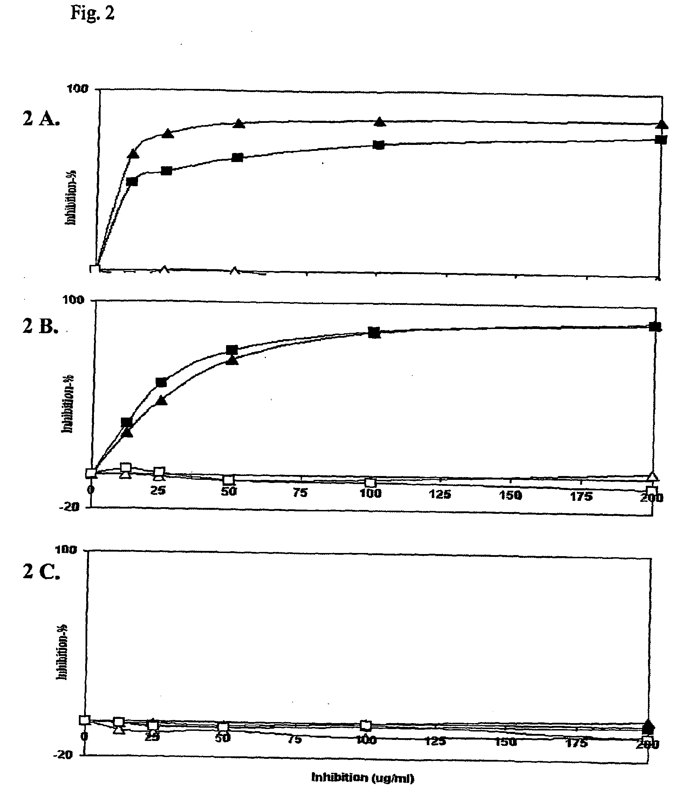

For detecting the antibody specificity we tested both type I and II collagen telopeptide assays and an anti-CCP Mark2 assay kit. Patient sera were diluted in assay buffer so that in each assay the initial binding could be noticeably inhibited with the respective peptides. Serial dilutions of competing peptides (arginine and citrulline forms of collagen C-telopeptides of α1(I) and α1(II)) were added (Figure 2). Inhibition by the soluble form of the same peptide as that coupled to the plate served as a specificity control in both telopeptide assays. For anti-CCP assay no such peptide was available for specificity testing. The percentage inhibition was plotted against the soluble peptide concentration. The signal (wavelength of 450 nm) obtained with the human serum only (initial binding) was defined as 0 % inhibition, and the signal of the blank (no serum sample) was defined as 100 % inhibition. We inhibited the binding with either the normal or the citrullinated antigen in soluble form. The soluble telopeptide antigens inhibited the binding in both type I and type II collagen telopeptide assays (Figure 2A and 2B).

Figure 2. Competition assays. One serum sample was tested in three different ELISAs: citrullinated carboxytelopeptide assays of the α1-chain of type I (A) and II collagens (B) and anti-CCP assays (C). The inhibitors were EKAHDGGRYYRA (SEQ ID NO:4), (open triangles), EKAHDGGRYYXA (SEQ ID NO:1), (closed triangles, corresponding to the immobilized antigen in the type I collagen assay), EKGPDPLQYMRA (SEQ ID NO:5), (open square) and EKGPDPLQYMXA(SEQ ID NO:3), (closed square, corresponding to the immobilized antigen in the type II collagen assay).

Thus it seems that identical or similar antibodies are involved in the binding. However, the collagen telopeptides bind a different antibody species than does the anti-CCP assay, since the anti-CCP binding cannot be inhibited with these peptides (Figure 2C).

Although the autoantibodies against collagen are different from those detected with the anti-CCP assay, both autoantibodies are increased in patients with rheumatoid arthritis. As shown in table 2, there was a significant correlation between these autoantibodies.

**Table 2.**

| Contingency coefficients between antibody binding to citrullinated C-telopeptides from type I (α1 and α2 chains) and type II (α1 chain) collagens and the anti-CCP assay results (based on sera from 120 rheumatoid arthritis patients). ^{***} p < 0.001 | | | | |
|---|---|---|---|---|
| | **anti-**α**1(I)** | **anti-**α**2(I)** | **anti-**α**1(II)** | **anti-CCP** assay |
| **anti-α1(I)** | | C = 0.380^{***} | C = 0.286^{***} | C = 0.310^{***} |
| | | p < 0.001 | p=0.001 | p < 0.001 |
| **anti-α2(I)** | C = 0.380^{***} | | C = 0.398^{***} | C = 0.112 |
| | p < 0.001 | | p < 0.001 | p=0.217 |
| **anti-α1(II)** | C = 0.286^{***} | C = 0.398^{***} | | C = 0.215^{*} |
| | p = 0.001 | p< 0.001 | | p = 0.016 |
| **anti-CCP assay** | | | C = 0.215^{***} | |
| | C = 0.310^{***} | C = 0.112 | p=0.016 | |
| | p < 0.001 | p = 0.217 | | |

### EXAMPLE 4

### Inhibition assay

Since there are also antibodies against normal type I and type II collagens in rheumatoid arthritis, for detecting specific antibodies against citrullinated forms two assays should be performed using both arginine and citrulline containing peptides of the type I or type II carboxytelopeptides. The absorbance of the reaction with arginine containing peptides is then subtracted from that of the reaction with the respective citrulline containing peptides, or the binding ratio of citrulline and arginine containing peptides is calculated.

Preferentially it is also possible to use only citrullinated peptide assay version, where this assay is performed with standard condition and with adding the same or similar soluble peptide in assay solution (200 µg/ml).

Sera from RA patients (n = 120) and controls (n = 80) were diluted 1:100 in assay buffer and/or inhibiting buffer (with soluble citrulline containing peptide (200 µg/ml)). The inhibition time was 30 minutes. After the inhibition reaction, the sera were moved to the wells of streptavidin-coated 96-well assay plates to which the biotinylated citrulline containing peptides had been coupled. After washing (3 times with PBS/0.05% [vol/vol] Tween-20), 100 µl of anti-human IgG conjugated to peroxidase (Product # 31412, Pierce, Rockford, IL, USA) diluted 1:7500 in EIA buffer (20mM Tris-HCl, 150mM NaCl, 0.1 % BSA, 0.05% Tween-20, pH 7.5) was added. After incubation for an hour at room temperature, the plates were washed (3 times with PBS/Tween-20). The bound antibodies were detected with 3,3'-5,5'-tetramethyl-benzidine (Sigma-Aldrich, St.Louis, MN, USA) as a substrate (0.01 mg/100 µl per well in 100 mM sodium acetate trihydrate, 1.5 mM citric acid monohydrate, 0.0015 % H₂O₂). After 30 minutes, the reaction was stopped by adding 100 µl 2M sulphuric acid/well. The plates were read at a wavelength of 450 nm in a Victor² instrument (Wallac, Turku, Finland). All the sera were tested in duplicate. The coefficients of variation were generally less than 10 %.

With type II collagen carboxytelopeptide in an ELISA experiment, the inhibition-% with the respective soluble antigen in controls was 5.4 % ± 3.8 (mean ± SD), and in RA patients 38 out of 120 showed increased inhibition (more inhibition than 13.0%).

Thus it is sufficient to make the assays with citrullinated forms of telopeptides, if in addition is confirmed that these autoantibodies can be inhibited with corresponding soluble antigens. This modification reduced the variance, since only one assay is used, and surprisingly more positives were found than with test using two ELISA assays.

## Claims

1. A method for detecting autoantibodies formed in relation to rheumatoid arthritis, which comprises the steps
- contacting a peptide or peptides with a biological fluid sample under suitable conditions for the formation of an immunological complex or complexes; and
- detecting and optionally measuring the amount of autoantibodies formed,
wherein the peptide is selected from the group comprising
- a peptide which comprises a sequence derived from a carboxyterminal telopeptide of α1 chain of type I collagen,
- a peptide which comprises a sequence derived from a carboxyterminal telopeptide of α2 chain of type I collagen,
- a peptide which comprises a sequence derived from a carboxyterminal telopeptide of α1 chain of type II collagen,
and wherein at least the last arginine residue in the peptide sequence is converted to citrulline.

2. The method according to claim 1, wherein the peptide(s) ends to a sequence selected from the group comprising-YYXA, -FYXA and -YN4XA, wherein X is citrulline.

3. The method according to claim 1 or 2, wherein the peptide(s) is / are isolated from tissue collagens and the arginine(s) is / are converted to citrulline(s) *in vitro.*

4. The method according to claim 1 or 2, wherein the peptide(s) is partly or fully a synthetic peptide.

5. The method according to any one of the preceding claims, wherein the length of the peptide(s) is 10 to 50, preferably 12 to 30 amino acids.

6. The method according to any one of the preceding claims, wherein the peptide(s) comprises a sequence selected from the group comprising EKAHDGGRYYXA (SEQ ID NO:1), YDFGYDGDFYXA (SEQ ID NO:2), and EKGPDPLQYMXA (SEQ ID NO:3), where X is citrulline.

7. The method according to any one of the preceding claims, wherein the biological fluid sample is a serum sample or a synovial fluid sample.

8. The method according to any one of the preceding claims, wherein the peptide(s) comprises a label selected from the group comprising a radioactive label, a luminescent label, a fluorescent label, a lanthanide, and an enzyme.

9. The method according to any one of the preceding claims, wherein the immunologic reaction between the peptide and the biological fluid sample is carried out in a solid phase and the peptide is bound directly or indirectly to the test tube or plate or a corresponding solid phase.

10. The method according to any one of the preceding claims, wherein the immunologic reaction between the peptide and the biological fluid sample is carried out in a liquid phase and the peptide sequence is bound directly or indirectly to solid particles in the liquid.

11. The method according to any one of the preceding claims, wherein the autoantibodies are detected or their amount measured as the intensity of the absorbance in an ELISA assay.

12. The method according to any one of the preceding claims, wherein the autoantibodies are detected or their amount measured as the emitted light in a chemiluminesence assay.

13. The method according to any one of the preceding claims, wherein the autoantibodies are detected or their amount measured so that bound citrulline peptide is compared to bound arginine peptide.

14. The method according to any one of the preceding claims, wherein the autoantibodies are detected or their amount measured in assay solution comprising soluble citrulline peptides.

15. A citrullinated peptide selected from the group comprising
- a peptide which comprises a sequence derived from a carboxyterminal telopeptide of α1 chain of type I collagen,
- a peptide which comprises a sequence derived from a carboxyterminal telopeptide of α2 chain of type I collagen,
- a peptide which comprises a sequence derived from a carboxyterminal telopeptide of α1 chain of type II collagen,
and wherein at least the last arginine residue in the peptide sequence is converted to citrulline.

16. The citrullinated peptide according to claim 15, wherein the peptide ends to a sequence selected from the group comprising-YYXA, -FYXA and -YMXA, wherein X is citrulline.

17. The citrullinated peptide according to claim 15 or 16, wherein the length of the peptide(s) is 10 to 50, preferably 12 to 30 amino acids.

18. The citrullinated peptide according to any one of claims 15 to 17, wherein the peptide sequence comprises a sequence selected from the group comprising EKAHDGGRYYXA (SEQ ID NO:1), YDFGYDGDFYXA (SEQ m NO:2), and EKGPDPLQYMXA (SEQ ID NO:3).

19. A kit comprising at least one citrullinated peptide according to any one of claims 15 to 18.

20. A pharmaceutical composition comprising an effective amount of at least one of the peptides according to any one of claims 15 to 18.

21. The pharmaceutical composition according to claim 20, wherein the composition is suitable for oral administration or injection.

22. The pharmaceutical composition according to claim 20 or 21, wherein the composition is to be administrated to a patient in the amount of 1- 50 µg/day, preferably 1- 25 µg/day.

## Patentansprüche

1. Verfahren zum Nachweis von Autoantikörpern gebildet in Bezug auf rheumatoide Arthritis, welches die Schritte umfasst
- Inkontaktbringen eines Peptids oder von Peptiden mit einer biologischen flüssigen Probe unter geeigneten Bedingungen für die Bildung eines immunologischen Komplexes oder immunologischer Komplexe; und
- Nachweis und gegebenenfalls Messung des Gehalts an gebildeten Auto-antikörpern, wobei das Peptid ausgewählt ist aus der Gruppe umfassend
- ein Peptid umfassend eine Sequenz, die abgeleitet ist von einem carboxyterminalen Telopeptid einer α1-Kette des Typ 1-Kollagens,
- ein Peptid umfassend eine Sequenz, die abgeleitet ist von einem carboxyterminalen Telopeptid einer α2-Kette des Typ 1-Kollagens,
- ein Peptid umfassend eine Sequenz, die abgeleitet ist von einem carboxyterminalen Telopeptid einer α1-Kette des Typ 11-Kollagens,
und wobei mindestens der letzte Argininrest in der Peptidsequenz zu Citrullin umgewandelt ist.

2. Verfahren nach Anspruch 1, wobei das oder die Peptid(e) mit einer Sequenz endet/enden, die ausgewählt ist aus der Gruppe umfassend -YYXA, -FYXA und -YMXA, wobei X Citrullin ist.

3. Verfahren nach Anspruch 1 oder 2, wobei das oder die Peptid(e) aus Gewebekollagenen isoliert wird/werden und das oder die Arginin(e) *in vitro* zu Citrullin umgewandelt wird/werden.

4. Verfahren nach Anspruch 1 oder 2, wobei das oder die Peptid(e) teilweise oder vollständig ein synthetische(s) Peptid(e) ist/sind.

5. Verfahren nach einem der vorhergehenden Ansprüche, wobei die Länge des Peptids oder der Peptide 10 bis 50, vorzugsweise 12 bis 30 Aminosäuren ist.

6. Verfahren nach einem der vorhergehenden Ansprüche, wobei das oder die Peptid(e) eine Sequenz umfasst/umfassen, die ausgewählt ist aus der Gruppe umfassend EKAHDGGRYYXA (SEQ ID NO: 1), YDFGYDGDFYXA (SEQ ID NO: 2) und EKGPDPLQYMXA (SEQ ID NO: 3), wobei X Citrullin ist.

7. Verfahren nach einem der vorhergehenden Ansprüche, wobei die biologische flüssige Probe eine Serumprobe oder eine Gelenkflüssigkeitsprobe ist.

8. Verfahren nach einem der vorhergehenden Ansprüche, wobei das oder die Peptid(e) eine Markierung umfasst/umfassen, die ausgewählt ist aus der Gruppe umfassend eine radioaktive Markierung, eine lumineszente Markierung, eine fluoreszierende Markierung, ein Lanthanid und ein Enzym.

9. Verfahren nach einem der vorhergehenden Ansprüche, wobei die immunologische Reaktion zwischen dem Peptid und der biologischen flüssigen Probe in einer festen Phase durchgeführt wird und das Peptid direkt oder indirekt an das/die Teströhrchen oder -platte oder eine entsprechende feste Phase gebunden ist.

10. Verfahren nach einem der vorhergehenden Ansprüche, wobei die immunologische Reaktion zwischen dem Peptid und der biologischen flüssigen Probe in einer flüssigen Phase durchgeführt wird und die Peptidsequenz direkt oder indirekt an feste Partikel in der Flüssigkeit gebunden ist.

11. Verfahren nach einem der vorhergehenden Ansprüche, wobei die Autoantikörper nachgewiesen werden oder ihr Gehalt gemessen wird als Intensität der Extinktion in einem ELISA-Assay.

12. Verfahren nach einem der vorhergehenden Ansprüche, wobei die Autoantikörper nachgewiesen werden oder ihr Gehalt gemessen wird als das emittierte Licht in einem Chemilumineszenz-Assay.

13. Verfahren nach einem der vorhergehenden Ansprüche, wobei die Autoantikörper nachgewiesen werden oder ihr Gehalt gemessen wird, indem das gebundene Citrullinpeptid mit dem gebundenen Argininpeptid verglichen wird.

14. Verfahren nach einem der vorhergehenden Ansprüche, wobei die Autoantikörper nachgewiesen werden oder ihr Gehalt gemessen wird in einer Testlösung, die lösliche Citrullinpeptide umfasst.

15. Citrulliniertes Peptid ausgewählt aus der Gruppe umfassend:
- ein Peptid umfassend eine Sequenz, die abgeleitet ist von einem carboxyterminalen Telopeptid einer α1-Kette des Typ 1-Kollagens,
- ein Peptid umfassend eine Sequenz, die abgeleitet ist von einem carboxyterminalen Telopeptid einer α2-Kette des Typ 1-Kollagens,
- ein Peptid umfassend eine Sequenz, die abgeleitet ist von einem carboxyterminalen Telopeptid einer α1-Kette des Typ 11-Kollagens,
und wobei mindestens der letzte Argininrest in der Peptidsequenz zu Citrullin umgewandelt ist.

16. Citrulliniertes Peptid nach Anspruch 15, wobei das Peptid mit einer Sequenz endet, die ausgewählt ist aus der Gruppe umfassend -YYXA, -FYXA und-YMXA, wobei X Citrullin ist.

17. Citrulliniertes Peptid nach Anspruch 15 oder 16, wobei die Länge des Peptids oder der Peptide 10 bis 50, vorzugsweise 12 bis 30 Aminosäuren ist.

18. Citrulliniertes Peptid nach einem der Ansprüche 15 bis 17, wobei das Peptid eine Sequenz umfasst, die ausgewählt ist aus der Gruppe umfassend EKAHDGGRYYXA (SEQ ID NO: 1), YDFGYDGDFYXA (SEQ ID NO: 2) und EKGPDPLQYMXA (SEQ ID NO: 3).

19. Kit, der mindestens ein citrulliniertes Peptid nach einem der Ansprüche 15 bis 18 umfasst.

20. Arzneimittel umfassend eine wirksame Menge von mindestens einem der Peptide nach einem der Ansprüche 15 bis 18.

21. Arzneimittel nach Anspruch 20, wobei die Zusammensetzung für orale Verabreichung oder Injektion geeignet ist.

22. Arzneimittel nach Anspruch 20 oder 21, wobei die Zusammensetzung in der Menge von 1 - 50 µg/Tag, vorzugsweise 1 - 25 µg/Tag, an einen Patienten zu verabreichen ist.

## Revendications

1. Procédé de détection d'auto-anticorps formés en relation avec une polyarthrite rhumatoïde, comprenant les étapes suivantes
- le contact d'un peptide ou de peptides avec un échantillon de fluide biologique dans des conditions appropriées pour former un ou plusieurs complexes immunologiques ; et
- la détection et facultativement la mesure de la quantité d'auto-anticorps formés,
dans lequel le peptide est choisi dans le groupe comprenant
- un peptide qui comprend une séquence dérivant d'un télopeptide carboxyterminal de la chaîne α1 du collagène de type I,
- un peptide qui comprend une séquence dérivant d'un télopeptide carboxyterminal de la chaîne α2 du collagène de type I,
- un peptide qui comprend une séquence dérivant d'un télopeptide carboxyterminal de la chaîne α1 du collagène de type II,
et dans lequel au moins le dernier résidu arginine dans la séquence peptidique est converti en citrulline.

2. Procédé selon la revendication 1, dans lequel le ou les peptides se termine(nt) par une séquence choisie dans le groupe comprenant -YYXA, -FYXA et - YMXA, où X est la citrulline.

3. Procédé selon la revendication 1 ou 2, dans lequel le ou les peptide(s) est/sont isolé(s) à partir de collagènes tissulaires et la ou les arginine(s) est/sont convertie(s) en citrulline(s) *in vitro.*

4. Procédé selon la revendication 1 ou 2, dans lequel le ou les peptide(s) est/sont partiellement ou totalement un peptide synthétique.

5. Procédé selon l'une quelconque des revendications précédentes, dans lequel la longueur du ou des peptides est de 10 à 50 acides aminés, de préférence de 12 à 30 acides aminés.

6. Procédé selon l'une quelconque des revendications précédentes, dans lequel le ou les peptide(s) comprend/comprennent une séquence choisie dans le groupe comprenant EKAHDGGRYYXA (SEQ ID NO : 1), YDFGYDGDFYXA (SEQ ID NO : 2) et EKGPDPLQYMXA (SEQ ID NO : 3), où X est la citrulline.

7. Procédé selon l'une quelconque des revendications précédentes, dans lequel l'échantillon de fluide biologique est un échantillon de sérum ou un échantillon de fluide synovial.

8. Procédé selon l'une quelconque des revendications précédentes, dans lequel le ou les peptide(s) comprend/comprennent un marqueur choisi dans le groupe comprenant un marqueur radioactif, un marqueur luminescent, un marqueur fluorescent, un lanthanide ou une enzyme.

9. Procédé selon l'une quelconque des revendications précédentes, dans lequel la réaction immunologique entre le peptide et l'échantillon de fluide biologique est mise en oeuvre sur une phase solide et le peptide est lié directement ou indirectement au tube à essai ou à la plaque de test ou à une phase solide correspondante.

10. Procédé selon l'une quelconque des revendications précédentes, dans lequel la réaction immunologique entre le peptide et l'échantillon de fluide biologique est mise en oeuvre en phase liquide et la séquence peptidique est liée directement ou indirectement à des particules solides dans le liquide.

11. Procédé selon l'une quelconque des revendications précédentes, dans lequel les auto-anticorps sont détectés ou leur quantité est mesurée sous la forme de l'intensité d'absorbance dans un dosage ELISA.

12. Procédé selon l'une quelconque des revendications précédentes, dans lequel les auto-anticorps sont détectés ou leur quantité est mesurée sous la forme de la lumière émise dans un essai de chimioluminescence.

13. Procédé selon l'une quelconque des revendications précédentes, dans lequel les auto-anticorps sont détectés ou leur quantité est mesurée de manière à ce qu'un peptide lié contenant une citrulline soit comparé à un peptide lié contenant une arginine.

14. Procédé selon l'une quelconque des revendications précédentes, dans lequel les auto-anticorps sont détectés ou leur quantité est mesurée dans une solution d'essai comprenant des peptides solubles contenant une citrulline.

15. Peptide contenant une citrulline choisi dans le groupe comprenant
- un peptide qui comprend une séquence dérivant d'un télopeptide carboxyterminal de la chaîne α1 du collagène de type I,
- un peptide qui comprend une séquence dérivant d'un télopeptide carboxyterminal de la chaîne α2 du collagène de type I,
- un peptide qui comprend une séquence dérivant d'un télopeptide carboxyterminal de la chaîne α1 du collagène de type II,
et dans lequel au moins le dernier résidu arginine dans la séquence peptidique est converti en citrulline.

16. Peptide contenant une citrulline selon la revendication 15, dans lequel le peptide se termine par une séquence choisie dans le groupe comprenant -YYXA, -FYXA et -YMXA, où X est la citrulline.

17. Peptide contenant une citrulline selon la revendication 15 ou 16, dans lequel la longueur du ou des peptides est de 10 à 50 acides aminés, de préférence de 12 à 30 acides aminés.

18. Peptide contenant une citrulline selon l'une quelconque des revendications 15 à 17, dans lequel la séquence peptidique comprend une séquence choisie dans le groupe comprenant EKAHDGGRYYXA (SEQ ID NO : 1), YDFGYDGDFYXA (SEQ ID NO : 2) et EKGPDPLQYMXA (SEQ ID NO : 3).

19. Trousse comprenant au moins un peptide contenant une citrulline selon l'une quelconque des revendications 15 à 18.

20. Composition pharmaceutique comprenant une quantité efficace d'au moins un des peptides selon l'une quelconque des revendications 15 à 18.

21. Composition pharmaceutique selon la revendication 20, dans laquelle la composition est appropriée pour être administrée par voie orale ou par injection.

22. Composition pharmaceutique selon la revendication 20 ou 21, dans laquelle la composition doit être administrée à un patient en une quantité allant de 1 à 50 µg/jour, de préférence de 1 à 25 µg/jour.


### SEQUENCE LISTING

<110> Procollagen Oy
<120> Method for detecting autoantibodies formed in rheumatoid arthritis
<130> PROC01PCT
<150> 20050814
   <151> 2005-08-11
<160> 10
<170> PatentIn version 3.3
<210> 1
   <211> 12
   <212> PRT
   <213> Homo sapiens
<220>
   <221> misc_feature
   <223> Xaa is citrulline
<400> 1
   Glu Lys Ala His Asp Gly Gly Arg Tyr Tyr Xaa Ala
   1 5 10
<210> 2
   <211> 12
   <212> PRT
   <213> Homo sapiens
<220>
   <221> misc_feature
   <223> Xaa is citrulline
<400> 2
   Tyr Asp Phe Gly Tyr Asp Gly Asp Phe Tyr Xaa Ala
   1 5 10
<210> 3
   <211> 12
   <212> PRT
   <213> Homo sapiens
<220>
   <221> MISC_FEATURE
   <223> Xaa is citrulline
<400> 3
   Glu Lys Gly Pro Asp Pro Leu Gln Tyr Met Xaa Ala
   1 5 10
<210> 4
   <211> 12
   <212> PRT
   <213> Homo sapiens
<220>
   <221> MISC_FEATURE
   <223> Xaa is citrulline
<400> 4
   Glu Lys Ala His Asp Gly Gly Arg Tyr Tyr Arg Ala
   1 5 10
<210> 5
   <211> 12
   <212> PRT
   <213> Homo sapiens
<400> 5
   Glu Lys Gly Pro Asp Pro Leu Gln Tyr Met Arg Ala
   1               5                   10
<210> 6
   <211> 14
   <212> PRT
   <213> Homo sapiens
<220>
   <221> MISC_FEATURE
   <223> Xaa is arginine/citrulline
<400> 6
   Ser Ala Phe Ala Gly Leu Gly Pro Xaa Glu Lys Gly Pro Asp
   1               5                   10
<210> 7
   <211> 11
   <212> PRT
   <213> Homo sapiens
<220>
   <221> MISC_FEATURE
   <223> Xaa is arginine/citrulline
<400> 7
   Leu Ala Gly Gln Xaa Gly Ile Val Gly Leu Pro
   1               5                   10
<210> 8
   <211> 12
   <212> PRT
   <213> Homo sapiens
<220>
   <221> MISC_FEATURE
   <223> Xaa is arginine/citrulline
<400> 8
   Gly Pro Met Gly Pro Xaa Gly Pro Pro Gly Pro Ala
   1               5                   10
<210> 9
   <211> 14
   <212> PRT
   <213> Homo sapiens
<400> 9
   Gly Pro Pro Gly Pro Gln Gly Leu Ala Gly Gln Arg Gly Glu
   1 5 10
<210> 10
   <211> 30
   <212> PRT
   <213> Homo sapiens
<220>
   <221> MISC_FEATURE
   <223> Xaa is arginine/citrulline
<400> 10
   Gly Pro Pro Gly Pro Gly Ile Asp Met Ser Ala Phe Ala Gly Leu Gly
   1 5 10 15
   Pro Arg Glu Lys Gly Pro Asp Pro Leu Gln Tyr Met Xaa Ala
   20 25 30